# EUROPÄISCHE PATENTSCHRIFT

(11) **EP 4 580 720 B1**
(45) Veröffentlichungstag und Bekanntmachung des Hinweises auf die Patenterteilung: **22.04.2026**
(21) Anmeldenummer: 23761799.8
(22) Anmeldetag: 22.08.2023
(51) Int. Cl.: A61M 25/00

(54) **MEDIZINISCHES INSTRUMENT**
MEDICAL INSTRUMENT
INSTRUMENT MÉDICAL

(30) Priorität: 31.08.2022 DE 102022121987
(43) Veröffentlichungstag der Anmeldung: 09.07.2025
(73) Patentinhaber: Gaiselmann, Thomas, 78667 Villingendorf (DE); Riek, Siegfried, 78628 Rottweil (DE); Frech, Gerhard, 78598 Königsheim (DE); Frech, Roland, 78598 Königsheim (DE); Bachmann, Karl-Heinz, 78667 Villingendorf (DE); Getsch, Arnold, 78532 Tuttlingen (DE)
(72) Erfinder: GAISELMANN, Thomas, 78667 Villingendorf (DE); RIEK, Siegfried, 78628 Rottweil (DE); BACHMANN, Karl-Heinz, 78667 Villingendorf (DE); GETSCH, Arnold, 78532 Tuttlingen (DE); FRECH, Josef, verstorben (DE)
(74) Vertreter: Westphal, Mussgnug & Partner Patentanwälte mbB
(86) Internationale Anmeldenummer: PCT/EP2023/073006
(87) Internationale Veröffentlichungsnummer: WO 2024/046810

(56) Entgegenhaltungen:
- WO-A2-2004/035110
- DE-A1- 102004 063 798
- DE-A1- 102005 044 468
- DE-A1- 102007 052 513

## Beschreibung

Die Erfindung betrifft ein medizinisches Instrument gemäß dem Oberbegriff des Patentanspruchs 1.

Bei vielen medizinischen Eingriffen wird eine gerade Hohlnadel, welche an der Spitze angeschliffen ist (im Folgenden auch als Kanüle bezeichnet), beispielsweise durch die Haut in ein Blutgefäß eingestochen, um Blut zu entnehmen oder Pharmaka einzuspritzen. Bei anderen Eingriffen wird die Kanüle durch die Haut in Körpergewebe oder Organe eingeführt, um beispielsweise Anästhetika zu injizieren oder Körperflüssigkeit zu entnehmen, zum Beispiel bei einer Zystenpunktion.

Bei der Anästhesie des Gebärmutterhalses wird bisher an mehreren Stellen beispielsweise umlaufend bei 9, 12 und 3 Uhr paracervikal, das heißt neben dem Gebärmutterhals, in das hintere Scheidengewölbe eingestochen, um die entsprechenden Nerven zu anästhesieren. Diese Einstiche sind schmerzhaft und es muss mehrfach eingestochen werden. Weiterhin können bis zum Erreichen des Zielgebietes Blutgefäße verletzt werden. Aus dem Gebärmutterhalskanal heraus kann der Einstich durch die Innenwand zum Zielgebiet schmerzfrei und ohne Verletzungspotenzial großer Gefäße erfolgen.

Die DE 10 2005 044 468 A1 offenbart ein medizinisches Instrument mit einem Führungsrohr, das in einen Körperkanal eines Patienten einführbar ist. Üblicherweise ist das Lumen des Körperkanals sehr begrenzt. Das Führungsrohr weist ein offenes proximales Ende und ein geschlossenes distales Ende sowie ausgehend von dem proximalen Ende einen inneren axialen Führungskanal auf, der in eine seitlich am Umfang des Führungsrohres angeordnete Öffnung mündet, so dass eine elastisch biegsame Kanüle durch das proximale Ende in den Führungskanal einführbar ist und beim Vorschieben in dem Führungskanal mit einer distalen Spitze gegen die Achse des Führungsrohres abgebogen seitlich durch die Öffnung austritt, wodurch der Eintrittswinkel der Kanüle in die Wand des Körperkanals vergrößert werden kann. Das Führungsrohr ist mit seinem proximalen Ende um seine Längsachse drehbar an einem Griffteil angeordnet ist, um bei gleicher Positionierung des medizinischen Instruments im Körperkanal verschiedene Einstichpunkte umlaufend erzeugen zu können.

Die Kanüle ist in der Regel aus einem harten Material wie beispielsweise Metall gefertigt. Wird das medizinische Instrument ebenfalls aus Metall gefertigt, entstehen hohe Fertigungskosten. Wird das medizinische Instrument, insbesondere das Führungsrohr, aus Kunststoff gefertigt, kann bei Vorschieben der distalen Spitze der Kanüle in dem Führungskanal und Ablenken zur seitlich angeordneten Öffnung der Führungskanal durch die distale Spitze beschädigt werden. Insbesondere besteht die Gefahr des Abschabens von Kunststoffpartikeln durch die distale Spitze, wobei die Kunststoffpartikel entweder die distale Spitze verstopfen können oder durch die seitlich angeordnete Öffnung in den Körperkanal austreten können.

Die Aufgabe der Erfindung besteht daher darin, ein medizinisches Gerät mit einem Führungsrohr, das in einen Körperkanal eines Patienten einführbar ist, derart weiterzuentwickeln, dass die Sicherheit bei der Anwendung verbessert wird.

Die Aufgabe wird erfindungsgemäß gelöst durch ein medizinisches Gerät mit den Merkmalen des Patentanspruchs 1.

Vorteilhafte Ausgestaltungen und Weiterbildungen sind in den abhängigen Ansprüchen angegeben.

Das erfindungsgemäße medizinisches Instrument mit einem Führungsrohr, das in einen Körperkanal eines Patienten einführbar ist, wobei das Führungsrohr ein offenes proximales Ende und ein geschlossenes distales Ende sowie ausgehend von dem proximalen Ende einen inneren axialen Führungskanal aufweist, der in eine seitlich am Umfang des Führungsrohres angeordnete Öffnung mündet, so dass eine elastisch biegsame Kanüle durch das proximale Ende in den Führungskanal einführbar ist und beim Vorschieben in dem Führungskanal mit einer distalen Spitze gegen die Längsachse des Führungsrohres abgebogen seitlich durch die Öffnung austritt, wobei das Führungsrohr um seine Längsachse drehbar an einem Griffteil angeordnet ist, wobei der Führungskanal einen ersten Abschnitt angrenzend an das proximale Ende und einen zweiten Abschnitt angrenzend an die Öffnung aufweist, wobei eine Wandung des Führungskanals in dem ersten, insbesondere proximalen, Abschnitt aus Kunststoff gefertigt ist und dadurch ausgezeichnet, dass eine Wandung in dem zweiten, insbesondere distalen, Abschnitt aus einem Material gefertigt ist, welches eine größere Härte als der Kunststoff des ersten Abschnitts aufweist. Die Gefahr der Beschädigung durch ein Entlangschaben oder Entlangkratzen der distalen Spitze an der Wandung des Führungskanals ist in dem Abschnitt angrenzend an die Öffnung besonders hoch. Der Erfindung liegt daher die Idee zugrunde, gerade diesen Abschnitt besonders abriebfest auszugestalten, insbesondere ohne das gesamte Führungsrohr aus einem Material entsprechender Härte zu fertigen.

Die Wandung kann beispielsweise durch eine entsprechende Beschichtung der Führungskanalinnenseite ausgebildet sein. Gemäß einer besonders bevorzugten Ausführungsform der Erfindung ist der Führungskanal in dem ersten Abschnitt gerade ausgebildet und in dem zweiten Abschnitt schräg oder gekrümmt ausgebildet. Die Gefahr der Beschädigung durch ein Entlangschaben oder Entlangkratzen der distalen Spitze an der Wandung des Führungskanals ist in dem gekrümmten Abschnitt, in welchem der Führungskanal von der Achse des Führungsrohrs zur seitlichen Öffnung abbiegt, besonders hoch, so dass gerade dieser Abschnitt des Führungskanals vorteilhafterweise eine Wandung aus einem Material aufweist, welches eine größere Härte als der Kunststoff des ersten Abschnitts aufweist.

Die Wandung kann beispielsweise durch eine entsprechende Beschichtung der Führungskanalinnenseite ausgebildet sein.

Besonders bevorzugt weist jedoch das Führungsrohr einen ersten Führungsrohrabschnitt und einen zweiten Führungsrohrabschnitt auf, wobei der erste Führungsrohrabschnitt den ersten Abschnitt des Führungskanals und der zweite Führungsrohrabschnitt den zweiten Abschnitt des Führungskanals umfasst, und wobei der erste Führungsrohrabschnitt aus Kunststoff und der zweite Führungsrohrabschnitt aus einem Material gefertigt ist, welches eine größere Härte als der Kunststoff des ersten Führungsrohrabschnitts aufweist. Mit anderen Worten gesagt ist ein distaler Endbereich des Führungsrohrs, in welchem sich insbesondere die seitliche Öffnung befindet, aus dem Material mit größerer Härte als der Kunststoff, aus welchem der verbleibende Teil des Führungsrohrs gefertigt ist, hergestellt. Insbesondere ist damit das Führungsrohr wenigstens zweiteilig ausgebildet.

Vorzugsweise ist das Material, welches eine größere Härte als der Kunststoff des ersten Abschnitts aufweist, Metall, insbesondere Edelstahl, besonders bevorzugt Medizinstahl. Diese Materialien haben sich als besonders abriebfest erwiesen, wobei insbesondere Medizinstahl für den Einsatz bei einem medizinischen Instrument entsprechend geeignet ist.

Gemäß einer besonders bevorzugten Ausführungsform der Erfindung ist der Kunststoff ein transparenter oder transluzenter Kunststoff. Dadurch wird einem Benutzer die Sicht auf die zumindest abschnittsweise in dem Führungsrohr angeordnete Spritze ermöglicht, wodurch die Bedienung des medizinischen Instruments vereinfacht und die Sicherheit erhöht werden kann.

Gemäß einer bevorzugten Ausführungsform der Erfindung weist das Führungsrohr an seinem proximalen Ende einen rohrförmigen Fixierabschnitt aufweist, der einen an dem Griffteil angeordneten rohrförmigen Befestigungsabschnitt drehbar gelagert übergreift. Durch eine derartige Ausgestaltung kann eine einfache und zuverlässige drehbar gelagerte Befestigung des Führungsrohrs ermöglicht werden.

Vorzugsweise weist das Griffteil eine Aufnahme für eine Spritze aufweist, in welcher die Spritze axial bewegbar ist zwischen einer proximalen Position, in welcher sich die distale Spitze ihrer Kanüle zurückgezogen in dem Führungskanal befindet, und einer distalen Position, in welcher die distale Spitze der Kanüle durch die Öffnung aus dem Führungsrohr austritt. Eine derartige Ausgestaltung ermöglicht eine vielseitige und besonders sichere Verwendbarkeit des Instruments. Insbesondere können handelsübliche Spritzen zum Einsatz kommen. In der proximalen Position der Spritze befindet sich die distale Spitze der Kanüle innerhalb des Führungskanals, so dass das Führungsrohr in den Körperkanal des Patienten eingeführt werden kann, ohne dass die Gefahr der Verletzung des Körperkanals durch die distale Spitze der Kanüle besteht. In der distalen Position der Spritze ist die Kanüle so weit vorgeschoben, dass ihre distale Spitze seitlich aus dem Führungsrohr austritt und durch die Wand des Körperkanals in das umliegende Gewebe in definiertem Austrittswinkel und definierter Austrittslänge einstechen kann.

Vorteilhafterweise weist die Aufnahme einen distalen Anschlag auf, der die axiale Bewegung der Spritze in distaler Richtung begrenzt, wobei die Spritze bei Anlage an dem distalen Anschlag in der distalen Position angeordnet ist und in dieser Position mittels eines ersten Rastmechanismus gehalten ist. In dieser Position kann der Anwender insbesondere die Sicherheit haben, dass das gewünschte Zielgebiet erreicht ist.

Vorzugsweise weist die Aufnahme einen proximalen Anschlag auf, der die axiale Bewegung der Spritze in proximaler Richtung begrenzt, wobei die Spritze bei Anlage an dem proximalen Anschlag in der proximalen Position angeordnet ist und in dieser Position mittels eines zweiten Rastmechanismus gehalten ist. Derartige Anschläge ermöglichen eine besonders einfache und reproduzierbare Handhabung des Instruments. Insbesondere kann der distale Anschlag die Länge definieren, um welche die distale Spitze der Kanüle aus dem Umfang des Führungsrohrs bzw. der Öffnung austritt, wodurch die Einstichtiefe der Kanüle in die Wand des Körperkanals des Patienten definiert werden kann. Auf diese Weise kann dem Anwender die Sicherheit gegeben werden, dass beim Einstellen der Einstichposition der Körperkanal nicht verletzt wird.

Ein Rastmechanismus zur fixierenden Halterung der Spritze bei Anlage an dem distalen und/oder proximalen Anschlag kann eine haptische Rückmeldung an den Benutzer über das Erreichen der jeweiligen Position geben und dadurch die Bediensicherheit weiter erhöhen.

Gemäß einer bevorzugten Ausführungsform der Erfindung ist an der Aufnahme wenigstens ein radial abstehender Flügel angebracht ist. Vorzugsweise sind zwei radial abstehende Flügel angebracht. Ein oder zwei derartige Flügel können dem Benutzer als Fingeranlagefläche dienen und die Handhabung des Instruments vereinfachen.

Falls gemäß einer vorteilhaften Weiterbildung der Erfindung an dem Griffteil ein Handgriff angeordnet ist, kann dies die Handhabung des Instruments weiter vereinfachen.

Weiterhin kann gemäß einer bevorzugten Ausführungsform der Erfindung an dem Führungsrohr ein auf dem Führungsrohr verschiebbarer Einstellring vorhanden sein, mittels welchem die Eindringtiefe des Führungsrohres in den Gebärmutterhalskanal definiert und somit die Anspritzpunkte individuell einstellbar gemacht werden können. Dazu kann eine Graduierung des Führungsrohres vorhanden sein.

Ein Ausführungsbeispiel der Erfindung wird anhand der nachfolgenden Figuren ausführlich erläutert. Es zeigen
- Figur 1: eine perspektivische Ansicht eines Ausführungsbeispiels eines erfindungsgemäßen medizinischen Instruments mit eingesetzter Spritze in einer distalen Position,
- Figur 2: einen Längsschnitt durch das Instrument gemäß Figur 1,
- Figur 3: eine Draufsicht auf das Instrument gemäß Figur 1,
- Figur 4: einen Längsschnitt durch das Instrument gemäß Figur 1 mit der Spritze in einer proximalen Position,
- Figur 5: eine Draufsicht auf das Instrument gemäß Figur 1 mit der Spritze in der proximalen Position,
- Figur 6: eine Ausschnittvergrößerung eines Querschnitts des distalen Endes des Instruments gemäß Figur 3 und
- Figur 7: eine Ausschnittvergrößerung eines Querschnitts des distalen Endes des Instruments gemäß Figur 5.

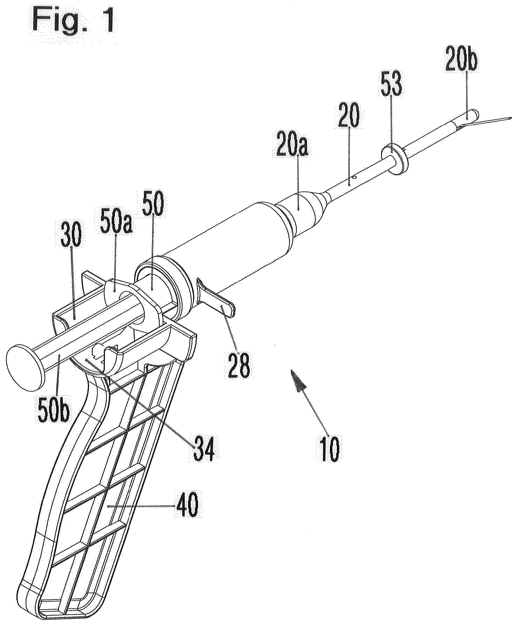

Die Figuren 1 bis 7 zeigen verschiedene Ansichten eines Ausführungsbeispiels eines erfindungsgemäßen medizinischen Instruments 10 mit einem Führungsrohr 20, das in einen Körperkanal eines Patienten einführbar ist. Das Führungsrohr 20 weist ein offenes proximales Ende 21 und ein geschlossenes distales Ende 22 auf. Das geschlossene distale Ende 22 kann dabei stumpf abgerundet ausgebildet und gegebenenfalls leicht konisch zulaufend ausgebildet sein. Das Führungsrohr 20 weist eine Längsachse A auf und ist insbesondere geradzylindrisch ausgebildet. Das Führungsrohr 20 weist ausgehend von dem proximalen Ende 21 einen inneren axialen Führungskanal 24 auf, der in eine seitlich am Umfang des Führungsrohres 20 angeordnete Öffnung 26 mündet. Die Öffnung 26 ist insbesondere in räumlicher Nähe zu dem geschlossenen distalen Ende 22 des Führungsrohrs 20 angeordnet. Der Führungskanal 24 weist einen ersten Abschnitt 25a angrenzend an das proximale Ende 21 und einem zweiten Abschnitt 25b angrenzend an die Öffnung 26 auf. Der Führungskanal 24 ist dabei insbesondere in dem ersten Abschnitt 25a gerade ausgebildet, während der Führungskanal 24 in dem zweiten Abschnitt 25b insbesondere schräg oder gekrümmt ausgebildet ist.

Der Führungskanal 24 weist eine Wandung 25 auf, wobei die Wandung 25 in dem ersten Abschnitt 25a aus Kunststoff gefertigt ist und die Wandung 25 in dem zweiten Abschnitt 25b aus einem Material gefertigt ist, welches eine größere Härte als der Kunststoff des ersten Abschnitts 25a aufweist.

Das Führungsrohr 20 kann einen ersten Führungsrohrabschnitt 20a und einen zweiten Führungsrohrabschnitt 20b aufweisen, wobei der erste Führungsrohrabschnitt 20a den ersten Abschnitt 25a des Führungskanals 24 und der zweite Führungsrohrabschnitt 20b den zweiten Abschnitt 25b des Führungskanals 24 umfassen kann, und wobei der erste Führungsrohrabschnitt 20a aus Kunststoff und der zweite Führungsrohrabschnitt 20b aus einem Material gefertigt ist, welches eine größere Härte als der Kunststoff des ersten Führungsrohrabschnitts 20a aufweist. Dadurch wird nicht lediglich die Wandung 25, sondern der entsprechend gesamte Abschnitt des Führungsrohrs 20 aus dem entsprechenden Material gefertigt, wodurch die Fertigung vereinfacht werden kann.

Das Material, welches eine größere Härte als der Kunststoff des ersten Abschnitts 25a aufweist, kann Metall, insbesondere Edelstahl, besonders bevorzugt Medizinstahl, sein. Der Kunststoff kann ein transparenter oder transluzenter Kunststoff sein.

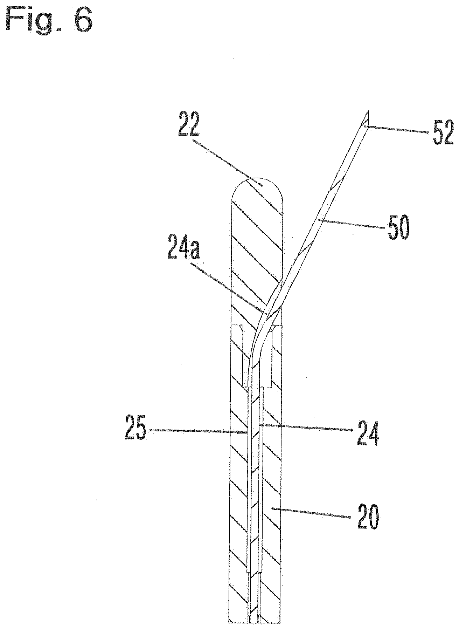

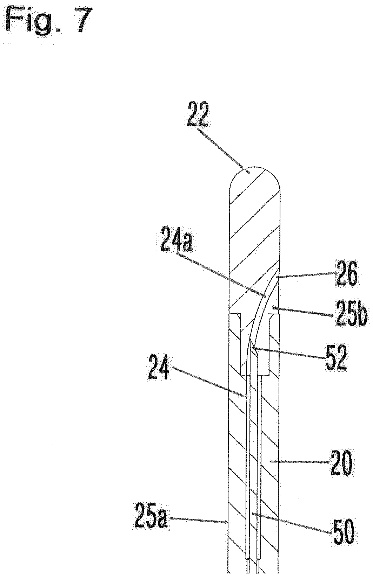

Aufgrund der seitlich angeordneten Öffnung 26 ist der Führungskanal 24 so ausgebildet, dass eine elastisch biegsame Kanüle 51 durch das proximale Ende 21 in den Führungskanal 24 einführbar ist und beim Vorschieben in dem Führungskanal 24 mit einer distalen Spitze 52 gegen die Längsachse A des Führungsrohres 20 abgebogen seitlich durch die Öffnung 26 austritt (vgl. insbesondere Figuren 6 und 7). Dazu kann insbesondere in dem Führungskanal 24 eine Ablenkeinrichtung 24a ausgebildet sein, beispielsweise in Form einer keilförmigen Schräge oder einer gekrümmten oder bogenförmig gerundeten Rampe, die vorzugsweise einen stufenlosen Übergang des geraden ersten Abschnitts 25a in die seitliche Öffnung bildet.

Der Durchmesser des Führungskanals 24 ist ausgehend von der Öffnung 26 insbesondere derart dimensioniert, dass die Kanüle 51 durch den Führungskanal 24 geführt geschoben werden kann. In distaler Richtung kann sich der Führungskanal 24 konisch aufweiten. Dabei kann die Länge des zwischen der Öffnung 26 und der konischen Aufweitung 24b angeordneten Teils des Führungskanals 24 auf die Länge der Kanüle 51 abgestimmt sein, wie nachfolgend noch näher erläutert wird. Die konische Aufweitung 24b kann als Einführhilfe zum Einführen der Kanüle 51 dienen. Anschließend an die konische Aufweitung 24b kann der Führungskanal 24 einen derart großen Durchmesser aufweisen, dass eine Spritze 50 zumindest abschnittsweise in dem Führungskanal 24 aufgenommen sein kann.

Das Führungsrohr 20 kann insbesondere mit seinem proximalen Ende 21 um seine Längsachse A drehbar an einem Griffteil 30 angeordnet sein. Dazu kann das Führungsrohr 20 an seinem proximalen Ende 21 einen rohrförmigen Fixierabschnitt 27 aufweisen, der einen an dem Griffteil 30 angeordneten rohrförmigen Befestigungsabschnitt 32 drehbar gelagert übergreift. Die axiale Befestigung kann dabei beispielsweise durch einen Rastmechanismus 29 erfolgen. Die drehbare Lagerung kann dadurch realisiert sein, dass der Rastmechanismus 29 über den Umfang umlaufend ausgebildet ist. Das Führungsrohr 20 kann lösbar an dem Griffteil 30 angeordnet sein, beispielsweise indem der Rastmechanismus 29 lösbar ausgebildet ist.

Der rohrförmige Fixierabschnitt 27 kann durch den Teil des Führungsrohrs 24 gebildet sein, in welchem die Spritze 50 abschnittsweise aufgenommen sein kann.

An dem Führungsrohr 20 kann ein Betätigungshebel 28 insbesondere radial nach außen abstehend angeordnet sein. Mittels des Betätigungshebels 28 kann das Führungsrohr 20 auf einfache Art und Weise um seine Längsachse A verdreht werden. Vorteilhafterweise ist der Betätigungshebel 28 in der gleichen Umfangswinkelposition wie die Öffnung 26 angeordnet, so dass der Betätigungshebel 28 die Winkelstellung der Öffnung 26 relativ zum Griffteil 30 anzeigen kann.

An dem Führungsrohr 20 kann ein auf dem Führungsrohr 20 verschiebbarer Einstellring 53 angeordnet sein, mittels welchem die Eindringtiefe des Führungsrohrs 20 definiert werden kann.

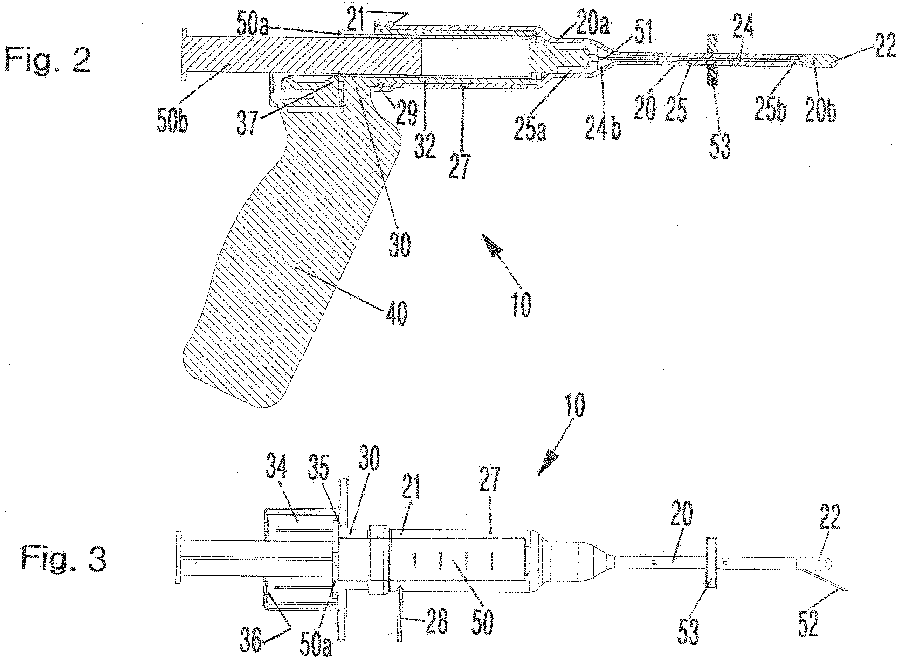

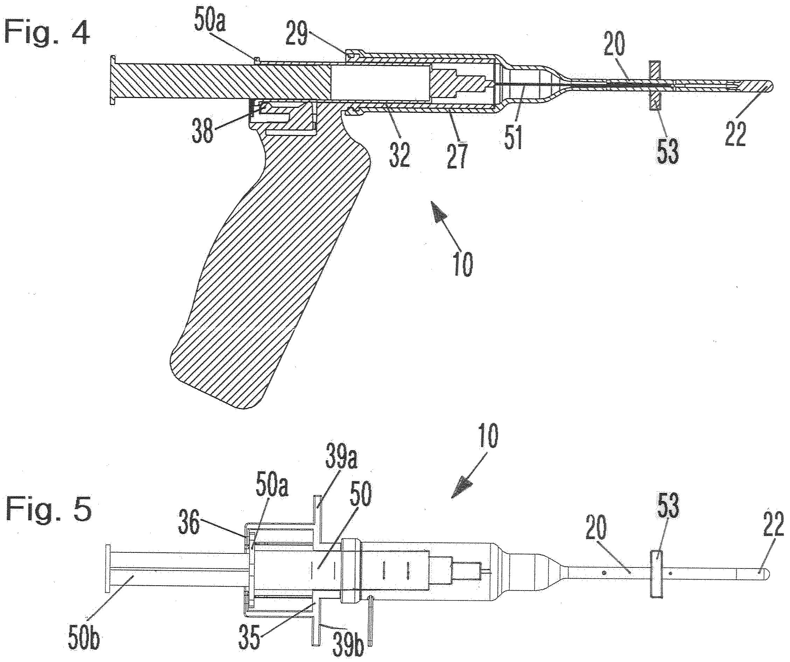

Das Griffteil 30 kann eine Aufnahme 34 für die Spritze 50 aufweisen, in welcher die Spritze 50 axial bewegbar ist zwischen einer proximalen Position, in welcher sich die distale Spitze 52 ihrer Kanüle 51 zurückgezogen in dem Führungskanal 24 befindet (vgl. Figuren 4, 5 und 7), und einer distalen Position, in welcher die distale Spitze 52 der Kanüle 51 durch die Öffnung 26 aus dem Führungsrohr 20 austritt (vgl. Figuren 2, 3 und 6). Die Aufnahme 34 kann beispielsweise halbschalenförmig und nach oben offen ausgebildet sein.

Die Spritze 50 kann in axialer Richtung von proximal in die Aufnahme 34 eingeführt werden, wobei insbesondere am proximalen Ende der Spritze 50 angeordnete Gegenhalteflügel 50a in der Aufnahme 34 zu liegen kommen. Insbesondere kann dabei das distale Ende der Spritze 50, an welcher die Kanüle 51 angeordnet ist, abschnittsweise in dem Führungsrohr 20, insbesondere in dem rohrförmigen Fixierabschnitt 27, aufgenommen sein.

Die Aufnahme 34 kann einen distalen Anschlag 35 aufweisen, der die axiale Bewegung der Spritze 50 in distaler Richtung begrenzt, wobei die Spritze 50 bei Anlage an dem distalen Anschlag 35 in der distalen Position angeordnet ist und in dieser Position mittels eines ersten Rastmechanismus 37 fixiert gehalten ist (vgl. insbesondere Figur 2). Der erste Rastmechanismus 37 kann beispielsweise als Vorsprung ausgebildet sein, der die Gegenhalteflügel 50a oder einen radial umlaufenden Bund am proximalen Ende der Spritze 50, welcher in die Gegenhalteflügel 50a übergeht, rastend hintergreift.

Die Aufnahme 34 einen proximalen Anschlag 36 aufweisen, der die axiale Bewegung der Spritze 50 in proximaler Richtung begrenzt, wobei die Spritze 50 bei Anlage an dem proximalen Anschlag 36 in der proximalen Position angeordnet ist und in dieser Position mittels eines zweiten Rastmechanismus 38 fixiert gehalten ist (vgl. insbesondere Figur 4). Der zweite Rastmechanismus 38 kann beispielsweise als Vorsprung ausgebildet sein, der die Gegenhalteflügel 50a oder den radial umlaufenden Bund am proximalen Ende der Spritze 50, welcher in die Gegenhalteflügel 50a übergeht, rastend hintergreift. Wenn einer der Gegenhalteflügel 50a der Spritze 50 an dem Rastmechanismus 38 anliegt, kann diese um 90 Grad gedreht werden, sodass eine Füllstandsmarkierung der Spritze 50 im transparenten Führungsrohr 20 einsehbar ist.

Der distale Anschlag 35 und der proximale Anschlag 36 können beispielsweise durch Kanten an den sich gegenüberliegenden Stirnflächen der Aufnahme 34 gebildet sein.

An der Aufnahme 34 kann wenigstens ein radial abstehender Flügel 39a angebracht sein. In dem vorliegenden Ausführungsbeispiel sind an der Aufnahme 34 zwei radial abstehende Flügel 39a, 39b angebracht, welche als Fingeranlagefläche dienen können.

An dem Griffteil 30, beispielsweise an der Unterseite der Aufnahme 34, kann ein Handgriff 40 angeordnet sein.

Das Griffteil 30 kann aus Kunststoff gefertigt sein. Insbesondere kann das Griffteil 30 einschließlich der Aufnahme 34 und des Handgriffs 40 einstückig gefertigt sein.

Wird das Instrument 10 zum Injizieren verwendet, wird in das Instrument 10 zunächst die Spritze 50 eingesetzt, beispielsweise eine passende handelsübliche Spritze 50, insbesondere in Form einer Einwegspritze 50 aus Kunststoff. Am distalen Ende der Spritze 50 wird die Kanüle 51 angesetzt. Die Kanüle 51 ist elastisch biegsam und beispielsweise aus Metall gefertigt. Die Spritze 50 umfasst einen axial verschiebbaren Stößel 50b, mittels welchem eine in der Spritze 50 angeordnete Flüssigkeit durch die Kanüle 51 ausgestoßen und injiziert werden kann.

Die Spritze 50 wird mit der aufgesetzten Kanüle 51 vom proximalen Ende her durch die Aufnahme 34 in das Führungsrohr 20 eingeführt. Die Kanüle 51 gelangt dabei insbesondere begünstigt durch die konische Aufweitung 24b in den distalen Teil des Führungskanals 24. Die Spritze 50 wird koaxial in der Aufnahme und dem proximalen Teil des Führungskanals 24, insbesondere in dem Fixierabschnitt 27, aufgenommen. Dabei kommt der umlaufende radiale Bund 50a der Spritze 50 insbesondere zwischen dem distalen Anschlag 35 und dem proximalen Anschlag 36 der Aufnahme 34 zu liegen.

Die axialen Längenabmessungen der Instruments 10 sind auf die Längenabmessungen der Spritze 50 und die Längenabmessungen der Kanüle 51 so abgestimmt, dass sich die distale Spitze 52 der Kanüle 51 innerhalb des Führungskanals 24 befindet, wenn der radiale Bund 50a der Spritze 50 an dem proximalen Anschlag 36 anliegt, und dass die distale Spitze 52 der Kanüle 51 um eine zum Injizieren definierte Länge aus der Öffnung 26 hervorsteht, wenn die Gegenhalteflügel 50a der Spritze 50 an dem distalen Anschlag 35 anliegen. Ist die Spritze 50 in der proximalen Position, kann das Instrument 10 gefahrlos in den Körperkanal des Patienten eingeführt werden. Wurde die gewünschte Position des Instruments 10 in dem Körperkanal erreicht, kann die Spritze 50 in die distale Position überführt werden, in welcher die distale Spitze 52 aus der Öffnung 25 hervorragt. Anschließend kann der Stößel 50b der Spritze axial bewegt werden, um die Flüssigkeit, beispielsweise ein Anästhetikum, zu injizieren. Nach einer Injektion in einer ersten Drehwinkelstellung kann die Spritze 50 in die proximale Position überführt werden und das Führungsrohr 20 kann mittels des Betätigungshebels 28 um seine Längsachse A verdreht werden, um in einer weiteren Drehwinkelstellung eine weitere Injektion nach erneuter Überführung der Spritze 50 in die distale Position vornehmen zu können.

### Bezugszeichenliste

- 10: Instrument
- 20: Führungsrohr
- 20a: Führungsrohrabschnitt
- 20b: Führungsrohrabschnitt
- 21: proximales Ende
- 22: distales Ende
- 24: Führungskanal
- 24a: Ablenkeinrichtung
- 24b: Aufweitung
- 25: Wandung
- 25a: erster Abschnitt
- 25b: zweiter Abschnitt
- 26: Öffnung
- 27: Fixierabschnitt
- 28: Betätigungshebel
- 29: Rastmechanismus
- 30: Griffteil
- 32: Befestigungsabschnitt
- 34: Aufnahme
- 35: distaler Anschlag
- 36: proximaler Anschlag
- 37: erster Rastmechanismus
- 38: zweiter Rastmechanismus
- 39a: Flügel
- 39b: Flügel
- 40: Handgriff
- 50: Spritze
- 50a: Gegenhalteflügel
- 50b: Stößel
- 51: Kanüle
- 52: distale Spitze
- 53: Einstellring
- A: Längsachse

## Patentansprüche

1. Medizinisches Instrument (10) mit einem Führungsrohr (20), das in einen Körperkanal eines Patienten einführbar ist, wobei das Führungsrohr (20) ein offenes proximales Ende (21) und ein geschlossenes distales Ende (22) sowie ausgehend von dem proximalen Ende (21) einen inneren axialen Führungskanal (24) aufweist, der in eine seitlich am Umfang des Führungsrohres (20) angeordnete Öffnung (26) mündet, so dass eine elastisch biegsame Kanüle (51) durch das proximale Ende (21) in den Führungskanal (24) einführbar ist und beim Vorschieben in dem Führungskanal (24) mit einer distalen Spitze (52) gegen die Längsachse (A) des Führungsrohres (20) abgebogen seitlich durch die Öffnung (26) austritt, wobei das Führungsrohr (20) um seine Längsachse (A) drehbar an einem Griffteil (30) angeordnet ist,
wobei der Führungskanal (24) einen ersten Abschnitt (25a) angrenzend an das proximale Ende (21) und einen zweiten Abschnitt (25b) angrenzend an die Öffnung (26) aufweist, wobei eine Wandung (25) des Führungskanals (24) in dem ersten Abschnitt (25a) aus Kunststoff gefertigt ist, **gekennzeichnet dadurch, dass** eine Wandung in dem zweiten Abschnitt (25b) aus einem Material gefertigt ist, welches eine größere Härte als der Kunststoff des ersten Abschnitts (25a) aufweist.

2. Instrument nach Anspruch 1,
**dadurch gekennzeichnet, dass** der Führungskanal (24) in dem ersten Abschnitt (25a) gerade ausgebildet ist und in dem zweiten Abschnitt (25b) schräg oder gekrümmt ausgebildet ist.

3. Instrument nach einem der vorhergehenden Ansprüche,
**dadurch gekennzeichnet, dass** das Führungsrohr (20) einen ersten Führungsrohrabschnitt (20a) und einen zweiten Führungsrohrabschnitt (20b) aufweist, wobei der erste Führungsrohrabschnitt (20a) den ersten Abschnitt (25a) des Führungskanals (24) und der zweite Führungsrohrabschnitt (20b) den zweiten Abschnitt (25b) des Führungskanals (24) umfasst, und wobei der erste Führungsrohrabschnitt (20a) aus Kunststoff und der zweite Führungsrohrabschnitt (20b) aus einem Material gefertigt ist, welches eine größere Härte als der Kunststoff des ersten Führungsrohrabschnitts (20a) aufweist.

4. Instrument nach einem der vorhergehenden Ansprüche,
**dadurch gekennzeichnet, dass** das Material, welches eine größere Härte als der Kunststoff des ersten Abschnitts (25a) aufweist, Metall, insbesondere Edelstahl, besonders bevorzugt Medizinstahl, ist.

5. Instrument nach einem der vorhergehenden Ansprüche,
**dadurch gekennzeichnet, dass** der Kunststoff ein transparenter oder transluzenter Kunststoff ist.

6. Instrument nach einem der vorhergehenden Ansprüche,
**dadurch gekennzeichnet, dass** das Führungsrohr (20) an seinem proximalen Ende (21) einen rohrförmigen Fixierabschnitt (27) aufweist, der einen an dem Griffteil (30) angeordneten rohrförmigen Befestigungsabschnitt (32) drehbar gelagert übergreift.

7. Instrument nach einem der vorhergehenden Ansprüche,
**dadurch gekennzeichnet, dass** das Griffteil (30) eine Aufnahme (34) für eine Spritze (50) aufweist, in welcher die Spritze (50) axial bewegbar ist zwischen einer proximalen Position, in welcher sich die distale Spitze (52) ihrer Kanüle (51) zurückgezogen in dem Führungskanal (24) befindet, und einer distalen Position, in welcher die distale Spitze (52) der Kanüle (51) durch die Öffnung (26) aus dem Führungsrohr (20) austritt.

8. Instrument nach Anspruch 7,
**dadurch gekennzeichnet, dass** die Aufnahme (34) einen distalen Anschlag (35) aufweist, der die axiale Bewegung der Spritze (50) in distaler Richtung begrenzt, wobei die Spritze (50) bei Anlage an dem distalen Anschlag (35) in der distalen Position angeordnet ist und in dieser Position mittels eines ersten Rastmechanismus (37) gehalten ist.

9. Instrument nach Anspruch 7 oder 8,
**dadurch gekennzeichnet, dass** die Aufnahme (34) einen proximalen Anschlag (36) aufweist, der die axiale Bewegung der Spritze (50) in proximaler Richtung begrenzt, wobei die Spritze (50) bei Anlage an dem proximalen Anschlag (36) in der proximalen Position angeordnet ist und in dieser Position mittels eines zweiten Rastmechanismus (38) gehalten ist.

10. Instrument nach einem der Ansprüche 7 bis 9,
**dadurch gekennzeichnet, dass** an der Aufnahme (34) wenigstens ein radial abstehender Flügel (39a) angebracht ist, vorzugsweise zwei radial abstehende Flügel (39a, 39b) angebracht sind.

11. Instrument nach einem der vorhergehenden Ansprüche,
**dadurch gekennzeichnet, dass** an dem Griffteil (30) ein Handgriff (40) angeordnet ist.

12. Instrument nach einem der vorhergehenden Ansprüche,
**dadurch gekennzeichnet, dass** an dem Führungsrohr (20) ein auf dem Führungsrohr (20) verschiebbarer Einstellring (53) angeordnet ist.

## Claims

1. Medical Instrument (10) with a guide tube (20) which is insertable into a body canal of a patient, wherein the guide tube (20) comprises an open proximal end (21) and a closed distal end (22) as well as, starting at the proximal end (21), an inner axial guide channel (24) which terminates into an opening (26) disposed laterally on the circumference of the guide tube (20) such that an elastically flexible cannula (51) can be inserted through the proximal end (21) into the guide channel (24) and when advanced in the guide channel (24) emerges with a distal tip (52) deflected with respect to the longitudinal axis (A) of the guide tube (20) laterally through the opening (26), wherein the guide tube (20) is disposed rotatably about its longitudinal axis (A) on a grip part (30), wherein the guide channel (24) comprises a first segment (25a) adjoining the proximal end (21) and a second segment (25b) adjoining the opening (26), wherein a wall (25) of the guide channel (24) in the first segment (25a) is fabricated of synthetic material, **characterized in that** a wall in the second segment (25b) is fabricated of a material with a greater hardness than the synthetic material of the first segment (25a).

2. Instrument as in Claim 1, **characterized in that** the guide channel (24) in the first segment (25a) is developed to be straight and in the second segment (25b) is developed to be oblique or curved.

3. Instrument as in one of the preceding Claims, **characterized in that** the guide tube (20) comprises a first guide tube segment (20a) and a second guide tube segment (20b), wherein the first guide tube segment (20a) comprises the first segment (25a) of the guide tube channel (24) and the second guide tube segment (20b) comprises the second segment (25b) of the guide tube channel (24), and wherein the first guide tube segment (20a) is fabricated of synthetic material and the second guide tube segment (20b) is fabricated of a material which has a greater hardness than the synthetic material of the first guide tube segment (20a).

4. Instrument as in one of the preceding Claims, **characterized in that** the material which has a greater hardness than the synthetic material of the first segment (25a) is metal, in particular special steel, especially preferably medical grade stainless steel.

5. Instrument as in one of the preceding claims, **characterized in that** the synthetic material is a transparent or translucent synthetic material.

6. Instrument according to one of the preceding Claims, **characterized in that** the guide tube (20) comprises at its proximal end (21) a tubular fixing segment (27) which engages behind a tubular securement segment (32) rotatably disposed on the grip part (30).

7. Instrument as in one of the preceding Claims, **characterized in that** the grip part (30) comprises a reception (34) for a syringe (50) in which the syringe (50) is axially movable between a proximal position, in which the distal tip (52) of its cannula (51) is located in retraction in the guide channel (24), and a distal position in which the distal tip (52) of the cannula (51) emerges from the guide tube (20) through the opening (26).

8. Instrument as in Claim 7, **characterized in that** the reception (34) comprises a distal abutment (35) that delimits the axial movement of the syringe (50) in the distal direction, wherein the syringe (50) when in contact on the distal abutment (35) is disposed in the distal position and in this position is secured by means of a first latching mechanism (37).

9. Instrument as in Claim 7 or 8, **characterized in that** the reception (34) comprises a proximal abutment (36) that delimits the axial movement of the syringe (50) in the proximal direction, wherein the syringe (50) when in contact on the proximal abutment (36) is disposed in the proximal position and in this position is secured by means of a second latching mechanism (38).

10. Instrument as in one of the Claims 7 to 9, **characterized in that** in the reception (34) at least one radially projecting lobe (39a) is disposed, preferably two radially projecting lobes (39a, 39b) are disposed.

11. Instrument as in one of the preceding Claims, **characterized in that** on the grip part (30) one handle (40) is disposed.

12. Instrument as in one of the preceding Claims, **characterized in that** on the guide tube (20) an adjustment ring (53) displaceable on the guide tube (20) is disposed.

## Revendications

1. Instrument (10) médical comprenant un tube (20) de guidage, qui peut être introduit dans un canal du corps d'un patient, dans lequel le tube (20) de guidage a une extrémité (21) proximale et une extrémité (22) distale, fermée, ainsi qu'à partir de l'extrémité (21) proximale, un canal (24) intérieur, axial de guidage, qui débouche dans une ouverture (26), disposée latéralement sur le pourtour du tube (20) de guidage, de manière à ce qu'une canule (51) pouvant fléchir élastiquement puisse être introduite dans le canal (24) de guidage en passant par l'extrémité (21) proximale, et, lorsqu'elle se déplace dans le canal (24) de guidage, sorte latéralement par l'ouverture (26) par une pointe (52) distale, en se courbant par rapport à l'axe (A) longitudinal du tube (20) de guidage, dans lequel le tube (20) de guidage est monté, tournant autour de son axe (A) longitudinal, sur une partie (30) de préhension,
dans lequel le canal (24) de guidage a un premier tronçon (25a) voisin de l'extrémité (21) proximale et un deuxième tronçon (25b) voisin de l'ouverture (26), dans lequel une paroi (25) du canal (24) de guidage dans le premier tronçon (25a) est en matière plastique,
**caractérisé en ce qu'**une paroi dans le deuxième tronçon (25b) est en un matériau, qui a une dureté plus grande que la matière plastique du premier tronçon (25a).

2. Instrument suivant la revendication 1,
**caractérisé en ce que** le canal (24) de guidage est constitué de manière rectiligne dans le premier tronçon (25a) et est constitué de manière inclinée ou incurvée dans le deuxième tronçon (25b).

3. Instrument suivant l'une des revendications précédentes,
**caractérisé en ce que** le tube (20) de guidage a un premier tronçon (20a) de tube de guidage et un deuxième tronçon (20b) de tube de guidage, dans lequel le premier tronçon (20a) de tube de guidage comprend le premier tronçon (25a) du canal (24) de guidage et le deuxième tronçon (20b) de tube de guidage le deuxième tronçon (25b) du canal (24) de guidage, et dans lequel le premier tronçon (20a) du tube de guidage est en matière plastique et le deuxième tronçon (20b) du tube de guidage est en un matériau, qui a une dureté plus grande que la matière plastique du premier tronçon (20a) du tube de guidage.

4. Instrument suivant l'une des revendications précédentes,
dans lequel le matériau qui a une dureté plus grande que la matière plastique du premier tronçon (25a) est du métal, en particulier, de l'acier fin, d'une manière particulièrement préférée, de l'acier pour la médecine.

5. Instrument suivant l'une des revendications précédentes,
**caractérisé en ce que** la matière plastique est une matière plastique transparente ou translucide.

6. Instrument suivant l'une des revendications précédentes,
**caractérisé en ce que** le tube (20) de guidage a, à son extrémité (21) proximale, un tronçon (27) tubulaire de fixation, qui recouvre, en étant monté tournant, un tronçon (32) tubulaire de fixation, monté sur la partie (30) de préhension.

7. Instrument suivant l'une des revendications précédentes,
**caractérisé en ce que** la partie (30) de préhension a un logement (34) pour une seringue (50), dans lequel la seringue (50) est mobile axialement entre une position proximale, dans laquelle la pointe (52) distale de sa canule se trouve en retrait dans le canal (24) de guidage, et une position distale, dans laquelle la pointe (52) distale de la canule (51) sort par l'ouverture (26) du tube (20) de guidage.

8. Instrument suivant la revendication 7,
**caractérisé en ce que** le logement (34) a une butée (35) distale, qui limite le déplacement axial de la seringue (50) dans la direction distale, dans lequel la seringue (50) est au contact de la butée (35) distale dans la position distale et est maintenue dans cette position au moyen d'un premier mécanisme (37) d'encliquetage.

9. Instrument suivant la revendication 7 ou 8,
**caractérisé en ce que** le logement (34) a une butée (36) proximale, qui limite le déplacement axial de la seringue (50) dans la direction proximale, dans lequel la seringue (50) est, lorsqu'elle est appliquée à la butée (36) proximale, dans la position proximale et est maintenue dans cette position au moyen d'un deuxième mécanisme (38) d'encliquetage.

10. Instrument suivant les revendications 7 à 9,
**caractérisé en ce que** sur le logement (34) est montée au moins une aile (39a) en saillie radialement, de préférence, deux ailes (39a, 39b) en saillie radialement.

11. Instrument suivant l'une des revendications précédentes,
**caractérisé en ce qu'**une poignée (40) est montée sur la partie (30) de préhension.

12. Instrument suivant l'une des revendications précédentes,
**caractérisé en ce que** sur le tube (20) de guidage est montée une bague (53) de réglage coulissant sur le tube (20) de guidage.
